# EUROPEAN PATENT APPLICATION

(11) **EP 4 265 638 A1**
(43) Date of publication of application: **25.10.2023**
(21) Application number: 21905330.3
(22) Date of filing: 29.10.2021
(51) Int. Cl.: C07K 16/28, C07K 19/00, C12N 15/13, C12N 15/62, C12N 15/867, C12N 5/10, A61K 39/00, A61P 35/02

(54) **CHIMERIC ANTIGEN RECEPTOR TARGETING CD123 AND USE THEREOF**

(30) Priority: 16.12.2020 CN 202011498660
(71) Applicant: Beijing Immunochina Pharmaceuticals Co., Ltd., Beijing 100195 (CN); Shandong Jinsai Co., Ltd., Yantai, Shandong 265400 (CN)
(72) Inventor: HE, Ting, Beijing 100195 (CN); QI, Feifei, Yantai, Shandong 265400 (CN); LU, Xin-An, Beijing 100195 (CN); DING, Yanping, Beijing 100195 (CN); LIANG, Mengmeng, Beijing 100195 (CN)
(74) Representative: BOVARD AG
(86) International application number: PCT/CN2021/127505
(87) International publication number: WO 2022/127401

(57) **Abstract**

Provided in the present invention is a single-chain antibody scFv that specifically binds to CD123, including an amino acid sequence selected from SEQ ID NOs: 56, 57, 58, 59, 60 and 61. Also provided is a chimeric antigen receptor targeting CD123, including an amino acid sequence selected from SEQ ID NOs: 65, 66, 67, 68, 69 and 70. Further provided are a nucleic acid molecule encoding the scFv or chimeric antigen receptor, a vector and cell containing the nucleic acid molecule, and the use of the antibody, chimeric antigen receptor, nucleic acid molecule, vector or cell in the preparation of a drug for treating tumor expressing CD123.

## Description

### TECHNICAL FIELD

The present invention relates to scFv sequences targeting CD123 having different antigen recognition domains, chimeric antigen receptors (CAR) and CAR-T cells designed based on these scFv sequences, and their use in the treatment of tumor expressing CD123, in particular acute myeloid leukemia.

### BACKGROUND

Acute myeloid leukemia (AML) is a malignant tumor with an increasing incidence that mainly affects the elderly with a median age of 66 years. The National Cancer Institute estimates that there will be 19,520 new cases in 2018, of which 10,670 may die of the disease [1]. Although the 12-month survival rate has increased from 20% to 30% in patients aged 65-74 years over the past three decades, the current therapeutic efficacy of AML is still unsatisfactory and these patients have a 5-year survival rate of <5% [2]. As for younger patients with AML, the main treatment method is intensive chemotherapy, as for patients at higher risk of recurrence (cure rate of 35-40%), allogeneic hematopoietic cell transplantation (AHCT) is used, while the prognosis for older patients remains poor [2]. Patients with relapsed AML have less than 50% chance of achieving a second relief, whereas only 16% of patients achieve disease control after the second rescue chemotherapy [3]. Since the introduction of combined chemotherapy of anthracyclines and cytarabine in the 1970s, it has been the main treatment method [4,5]. Therefore, there is a need for the new therapeutic strategies for the treatment of this disease.

The concept of chimeric antigen receptor T cells (CAR-T cells) was first proposed in the late 1980s, which combines antibody specificity with T cell killing effects to form an adoptive immune effect [6,7]. The molecular structure of chimeric antigen receptor (CAR) mainly includes antibody single-chain variable fragment (scFv), intracellular signal domain, and hinge region and transmembrane region connecting scFv and intracellular signal domain. In recent years, anti-CD 19 and anti-CD20 chimeric antigen receptor T cells (CAR-T cells) have been used to treat lymphoid malignancies (B-cell acute lymphoblastic leukemia and non-Hodgkin lymphoma) with good results [8]. At present, CAR-T targets against AML treatment include CD123, CD33, CLL1, FLT3, etc., wherein, there are the most studies regarding CAR-T targeting CD123.

CD123 is the IL-3 receptor α-chain (IL-3Rα). CD123, together with CD131, forms a high-affinity IL-3 receptor that promotes cell proliferation and differentiation while inhibiting apoptosis of hematopoietic cells when binds to IL-3 [9,10]. The high expression of CD123 is associated with the activation of signal transduction and transcriptional activator 5 (STATS) signalling pathway. The STATS activation pathway is involved in a variety of malignant blood disorders [11]. CD123 is primarily expressed in hematopoietic systems, and CD123 is lowly expressed or unexpressed in normal CD34⁺CD38⁻ cells [12]. In addition, under normal condition, CD123 is highly expressed in myeloid progenitor cells and B lymphatic progenitor cells, while CD123 is lowly expressed or unexpressed in erythroid progenitor cells and multidirectional differentiation potential progenitor cells [13,14]. CD123 is highly expressed in more than 60%-90% AML primary cells [15-18]. Bras AE et al. [19] tested 455 patients with AML, of which approximately 55% of patients with AML cells expressed CD123, with a positive rate of more than 80%.

Although CAR-T cells have achieved good results in tumor cell therapy, there are still major problems in the efficacy and safety of CAR-T, mainly such as off-target effects, cytokine storms, and neurotoxicity and so on [20]. The optimal efficacy and safety of CAR-T cells depends on a number of factors, wherein the effective activation of CAR-T cells plays an important role. The degree of activation of CAR-T cells is affected by a number of factors, including the affinity of CAR molecule scFv, the length of the hinge region, the co-stimulation signal domain, and the antigen density on the surface of the target cells. The scFv of the CAR molecule is used as a region that directly binds to the antigen on the surface of the target cells, and its affinity is of great significance to the activation of the overall CAR-T cells. When the affinity of the CAR molecule scFv is low, the CAR-T cells cannot get enough antigen recognition, so that the optimal activation state cannot be achieved; when the affinity of the CAR molecule scFv is high, CAR-T cells may be able to recognize normal cells with relatively low antigen density while recognizing tumor cells, resulting in a serious off-target effect.

Therefore, in order to further improve the safety and efficacy of CAR-T cells, ensure the effective activation of CAR-T cells, and avoid off-target effects, there is still a need to screen scFv with moderate affinity for the target.

### SUMMARY OF THE INVENTION

In the first aspect, the present invention provides an antibody that specifically binds to CD123, comprising a heavy chain variable domain VH and a light chain variable domain VL, wherein the heavy chain variable domain comprises a VH-CDR1 amino acid sequence as shown in SEQ ID NO: 1 (GYX₁X₂X₃X₄(D)YX₅X₆X₇), a VH-CDR2 amino acid sequence as shown in SEQ ID NO: 2 (X₈X₉X₁₀(X₁₁)X₁₂X₁₃X₁₄X₁₅X₁₆X₁₇X₁₈NX₁₉X₂₀X₂₁KX₂₂X₂₃X₂₄) and a VH-CDR3 amino acid sequence as shown in SEQ ID NO: 3 (AX₂₅X₂₆X₂₇X₂₈X₂₉X₃₀X₃₁(YD)X₃₂X₃₃X₃₄X₃₅X₃₆X₃₇X₃₈X₃₉X₄₀); wherein X₁ is selected from S and T; X₂ is selected from F and I; X₃ is selected from T, S and M; X₄ is selected from S, D, T and G; X₅ is selected from W, Y, V, A and N; X₆ is selected from M, V, I and W; X₇ is selected from N and H; X₈ is selected from Y and R; X₉ is selected from I and C; X₁₀ is selected from D, N, Y and S; X₁₁ is selected from P and C; X₁₂ is selected from Y and G; X₁₃ is selected from D, N and S; X₁₄ is selected from S, N, D and G; X₁₅ is selected from E, A, G and S; X₁₆ is selected from T, I and S; X₁₇ is selected from H, S and N; X₁₈ is selected from Y and S; X₁₉ is selected from Q, E and P; X₂₀ is selected from K, N and S; X₂₁ is selected from F and L; X₂₂ is selected from D, G and S; X₂₃ is selected from K and R; X₂₄ is selected from A and I; X₂₅ is selected from R and G; X₂₆ is selected from G, S and E; X₂₇ is selected from E, P and R; X₂₈ is selected from G and S; X₂₉ is selected from N, Y, L and W; X₃₀ is selected from W, Y and L; X₃₁ is selected from G and D; X₃₂ is selected from R and E; X₃₃ is selected from Y, S and T; X₃₄ is selected from D, Y and G; X₃₅ is selected from G and Y; X₃₆ is selected from Y and L; X₃₇ is selected from A, G and P; X₃₈ is selected from M and L; X₃₉ is selected from D and A; and X₄₀ is selected from Y and C;
wherein the light chain variable domain comprises a VL-CDR1 amino acid sequence as shown in SEQ ID NO: 4 (X₄₁SX₄₂X₄₃X₄₄X₄₅X₄₆X₄₇X₄₈X₄₉(QK)X₅₀X₅₁X₅₂X₅₃W), a VL-CDR2 amino acid sequence as shown in SEQ ID NO: 5 (X₅₄SX₅₅X₅₆X₅₇X₅₈X₅₉) and a VL-CDR3 amino acid sequence as shown in SEQ ID NO: 6 (X₆₀X₆₁X₆₂X₆₃X₆₄(P)X₆₅TF); wherein X₄₁ is selected from A and S; X₄₂ is selected from Q, K and S; X₄₃ is selected from S and D; X₄₄ is selected from I, V and L; X₄₅ is selected from S, N, D and L; X₄₆ is selected from K, Y, S, D and N; X₄₇ is selected from D and S; X₄₈ is selected from G and S; X₄₉ is selected from D and N; X₅₀ is selected from S and N; X₅₁ is selected from D and Y; X₅₂ is selected from L, I and M; X₅₃ is selected from A, N and H; X₅₄ is selected from G, T and A; X₅₅ is selected from T, N and Q; X₅₆ is selected from L, S and R; X₅₇ is selected from Q, E, A, I and D; X₅₈ is selected from S and P; X₅₉ is selected from G and E; X₆₀ is selected from Q and N; X₆₁ is selected from H, Y, S, F and G; X₆₂ is selected from N, D, H and Y; X₆₃ is selected from K, L, E, R and S; X₆₄ is selected from Y, L, D, S, F and T; and X₆₅ is selected from Y, R and W; preferably, the antibody is scFv.

In the second aspect, the present invention provides a chimeric antigen receptor targeting CD123 comprising: the antibody of the first aspect; hinge region; transmembrane domain; co-stimulation domain; and CD3ζ intracellular signal transduction domain.

In the third aspect, the present invention provides a nucleic acid molecule comprising a nucleotide sequence encoding the antibody of the first aspect or the chimeric antigen receptor of the second aspect.

In the fourth aspect, the present invention provides a vector comprising the nucleic acid molecule of the third aspect.

In the fifth aspect, the present invention provides a cell which is transfected with the vector of the fourth aspect, thereby comprising the nucleic acid molecule of the third aspect or the vector of the fourth aspect.

In the sixth aspect, the present invention provides a pharmaceutical composition comprising the antibody of the first aspect, the chimeric antigen receptor of the second aspect, the nucleic acid molecule of the third aspect, the vector of the fourth aspect or the cell of the fifth aspect, and a pharmaceutically acceptable carrier.

In the seventh aspect, the present invention provides the use of the antibody of the first aspect, the chimeric antigen receptor of the second aspect, the nucleic acid molecule of the third aspect, the vector of the fourth aspect or the cell of the fifth aspect in the preparation of a drug for treating tumor expressing CD123.

The present invention obtained the best scFv sequence of the target CD123 by performing affinity screening for scFv of CAR molecule of the target CD123 and comparison of killing effect for CAR-T cells, so that CAR-T cells have better safety and efficacy. In comparison to the prior art, the present invention can effectively improve the killing efficiency of CAR-T cells targeting CD123 against tumor cell lines and primary tumor cells, and improve cell proliferation ability.

### BRIEF DESCRIPTION OF THE DRAWINGS

Fig. 1 shows the transduction efficiency of CD123 CAR molecules of different scFv in T cells.
Fig. 2 shows the total cell proliferation of CD123 CAR-T during culture.
Fig. 3 shows the effect of target cells AML primary cells and THP-1 cells on CD123 CAR-T cell differentiation under stimulation, respectively.
Fig. 4 shows the killing efficiency of CD123 CAR-T against CD123-positive cell lines (THP-1) and CD123-negative cell lines (K562).
Fig. 5 shows the killing efficiency of CD123 CAR-T against AML primary cells.
Fig. 6 shows cytokine expression of CD123 CAR-T after AML primary cells are stimulated for 5h.

### DETAILED DESCRIPTION OF THE INVENTION

The present invention provides an antibody that specifically binds to CD123, comprising a heavy chain variable domain VH and a light chain variable domain VL, wherein the heavy chain variable domain comprises VH-CDR1, VH-CDR2 and VH-CDR3, and the light chain variable domain comprises VL-CDR1, VL-CDR2 and VL-CDR3.

In one specific embodiment, the VH-CDR1 comprises an amino acid sequence selected from the group consisting of SEQ ID NOs: 8, 9, 10, 11 and 12; the VH-CDR2 comprises an amino acid sequence selected from the group consisting of SEQ ID NOs: 14, 15, 16, 17 and 18; the VH-CDR3 comprises an amino acid sequence selected from the group consisting of SEQ ID NOs: 20, 21, 22, 23 and 24; the VL-CDR1 comprises an amino acid sequence selected from the group consisting of SEQ ID NOs: 26, 27, 28, 29 and 30; the VL-CDR2 comprises an amino acid sequence selected from the group consisting of SEQ ID NOs: 32, 33, 34, 35 and 36; and the VL-CDR3 comprises an amino acid sequence selected from the group consisting of SEQ ID NOs: 38, 39, 40, 41 and 42.

In particular, the antibody of the present invention that specifically binds to CD123 comprises:
1) a heavy chain variable domain containing VH-CDR1 as shown in SEQ ID NO: 8, VH-CDR2 as shown in SEQ ID NO: 14, and VH-CDR3 as shown in SEQ ID NO: 20; and a light chain variable domain containing VL-CDR1 as shown in SEQ ID NO: 26, VL-CDR2 as shown in SEQ ID NO: 32, and VL-CDR3 as shown in SEQ ID NO: 38; and/or
2) a heavy chain variable domain containing VH-CDR1 as shown in SEQ ID NO: 9, VH-CDR2 as shown in SEQ ID NO: 15, and VH-CDR3 as shown in SEQ ID NO: 21; and a light chain variable domain containing VL-CDR1 as shown in SEQ ID NO: 27, VL-CDR2 as shown in SEQ ID NO: 33, and VL-CDR3 as shown in SEQ ID NO: 39; and/or
3) a heavy chain variable domain containing VH-CDR1 as shown in SEQ ID NO: 10, VH-CDR2 as shown in SEQ ID NO: 16, and VH-CDR3 as shown in SEQ ID NO: 22; and a light chain variable domain containing VL-CDR1 as shown in SEQ ID NO: 28, VL-CDR2 as shown in SEQ ID NO: 34, and VL-CDR3 as shown in SEQ ID NO: 40; and/or
4) a heavy chain variable domain containing VH-CDR1 as shown in SEQ ID NO: 11, VH-CDR2 as shown in SEQ ID NO: 17, and VH-CDR3 as shown in SEQ ID NO: 23; and a light chain variable domain containing VL-CDR1 as shown in SEQ ID NO: 29, VL-CDR2 as shown in SEQ ID NO: 35, and VL-CDR3 as shown in SEQ ID NO: 41; and/or
5) a heavy chain variable domain containing VH-CDR1 as shown in SEQ ID NO: 12, VH-CDR2 as shown in SEQ ID NO: 18, and VH-CDR3 as shown in SEQ ID NO: 24; and a light chain variable domain containing VL-CDR1 as shown in SEQ ID NO: 30, VL-CDR2 as shown in SEQ ID NO: 36, and VL-CDR3 as shown in SEQ ID NO: 42.

In another specific embodiment, the heavy chain variable domain in the antibody of the present invention comprises an amino acid sequence selected from the group consisting of SEQ ID NOs: 44, 45, 46, 47 and 48, or an amino acid sequence having at least 85%, at least 90%, at least 95%, at least 99% identity thereto; and/or the light chain variable domain comprises an amino acid sequence selected from the group consisting of SEQ ID NOs: 50, 51, 52, 53 and 54, or an amino acid sequence having at least 85%, at least 90%, at least 95%, at least 99% identity thereto.

In particular, the present invention provides an antibody that specifically binds to CD123, comprising:
1) a heavy chain variable domain as shown in SEQ ID NO: 44 and a light chain variable domain as shown in SEQ ID NO: 50; and/or
2) a heavy chain variable domain as shown in SEQ ID NO: 45 and a light chain variable domain as shown in SEQ ID NO: 51; and/or
3) a heavy chain variable domain as shown in SEQ ID NO: 46 and a light chain variable domain as shown in SEQ ID NO: 52; and/or
4) a heavy chain variable domain as shown in SEQ ID NO: 47 and a light chain variable domain as shown in SEQ ID NO: 53; and/or
5) a heavy chain variable domain as shown in SEQ ID NO: 48 and a light chain variable domain as shown in SEQ ID NO: 54.

In another specific embodiment, the present invention provides an antibody that specifically binds to CD123 comprising an amino acid sequence selected from the group of SEQ ID NOs: 56, 57, 58, 59, 60 and 61, or an amino acid sequence having at least 85%, at least 90%, at least 95%, at least 99% identity thereto.

Preferably, the antibody of the present invention is a single-chain antibody scFv; more preferably, the heavy chain variable domain and the light chain variable domain of the single-chain antibody scFv are linked by a linker peptide chain rich in glycine and serine, and the order of the heavy chain variable domain and the light chain variable domain can be interchanged.

The present invention further provides a chimeric antigen receptor targeting CD123, comprising: the antibody according to the present invention; hinge region; transmembrane domain; co-stimulation domain; and CD3ζ intracellular signal transduction domain.

In a specific embodiment, the co-stimulation domain is selected from CD27, CD28, 4-1BB, OX-40, CD30, CD40, PD-1, ICOS, LFA-1, CD-2, CD7, LIGHT, NKG2C, B7-H3, or any combination thereof; preferably, the co-stimulation domain is selected from 4-1BB and CD28, and the 4-1BB comprises an amino acid sequence as shown in SEQ ID NO: 62.

In one specific embodiment, the CD3ζ intracellular signal transduction domain comprises an amino acid sequence as shown in SEQ ID NO: 63.

In one specific embodiment, the hinge region and the transmembrane domain are selected from IgG1, IgG4, CD8α, CD28, IL-2 receptor, IL-7 receptor, IL-11 receptor, PD-1 or CD34 hinge region and transmembrane domain.

Preferably, the chimeric antigen receptor comprises an amino acid sequence selected from the group consisting of SEQ ID NOs: 65, 66, 67, 68, 69 and 70, or an amino acid sequence having at least 85%, at least 90%, at least 95%, at least 99% identity thereto.

The present invention further provides a nucleic acid molecule comprising a nucleotide sequence encoding the antibody or the chimeric antigen receptor of the present invention.

The present invention further provides a vector comprising a nucleic acid molecule of the present invention; preferably, the vector is a viral vector; more preferably, the vector is a lentiviral vector.

The present invention further provides a cell which is transfected with the vector of the present invention, thereby comprising the nucleic acid molecule or the vector of the present invention; preferably, the cell is T cell.

The present invention further provides the use of the antibody, the chimeric antigen receptor, the nucleic acid molecule, the vector or the cell of the present invention in the preparation of a drug for treating tumor expressing CD123; preferably, the tumor expressing CD123 is acute myeloid leukemia (AML).

Unless otherwise specified, the technical solutions described herein may be any combination.

The present invention is further illustrated by the following examples.

### Example 1: CD123 CAR molecular design and CAR-T preparation

The present example took the design of CD123 CAR molecules having different antigen recognition domains, and CAR-T cell preparation as an example.

The principle for the design of the antigen recognition domain was as follows: CAR-T-1 was taken as a control group, wherein SCFv sequence of CD123 was reported by the University of Pennsylvania [21], which was named scFv-01 herein. CAR-T-2~CAR-T-7 were taken as modified groups, wherein the scFv of CAR-T-2-CAR-T-7 were derived from the monoclonal antibody sequence obtained from the mouse immune CD123 extracellular fusion protein, which were named scFv-02-07 herein.

The nucleotide sequences of CD123 scFv of the different antigen recognition domains were synthesized by the method of gene synthesis, and then the nucleotide sequences of CD123 scFv were linked to the sequences of CD8α hinge region, CD8α transmembrane region, 4-1BB and CD3ζ intracellular region by homologous recombination method to form a complete CAR molecule, i.e., CAR1-7. This CAR nucleic acid fragment was constructed into the lentiviral transfer plasmid pLenti6.3/V5 (Thermo Fisher, Waltham, MA, USA) by restriction endonuclease digestion and ligase ligation. HEK293T cell was transfected with lentiviral packaging plasmids pLP/VSVG, pLP1/MDK, pLP2/RSK (Thermo Fisher, Waltham, MA, USA) and the above obtained transfer plasmid using Lipofectamine 3000 (Thermo Fisher, Waltham, MA, USA), the medium was collected after 48 h, cell debris was removed by centrifugation at 300 g, centrifugation was performed with an ultracentrifuge at 25,000 rpm for 3 h. The pellet was dissolved with 1 mL of normal saline, i.e., obtaining the desired lentiviral vector.

T cells were isolated from peripheral blood mononuclear cells in healthy volunteers using CD3/CD28 beads (Thermo Fisher, Cat #40203D). The isolated and purified T cells at 1.0 ×10⁶ cells/mL, were seeded to X-VIVO 15 medium (Lonza, Switzerland) containing 500 IU/mL IL-2 (Shandong Jintai Bioengineering Co., Ltd., China) for culture. After 48 h of culture, T cells were infected with a lentiviral vector carrying CD123 CAR molecules at MOI=0.5 to obtain CAR-T-1-7. After lentiviral infection for 24h, cell fluid exchange was performed and the lentivirus was seeded to fresh culture system X-VIVO 15 plus 500 IU/mL IL-2 for continuous culture. After lentiviral infection for 5 days, the cells in the culture system were repeatedly blown using a pipette and collected into a centrifuge tube, where CD3/CD28 beads were removed on a magnetic stand. T cells were centrifuged and counted, and some cells were taken and detected using flow cytometry (NovoCyte 2060R, ACEA Biosciences, San Diego, CA, USA) for the CAR molecular transduction efficiency of each group of cells.

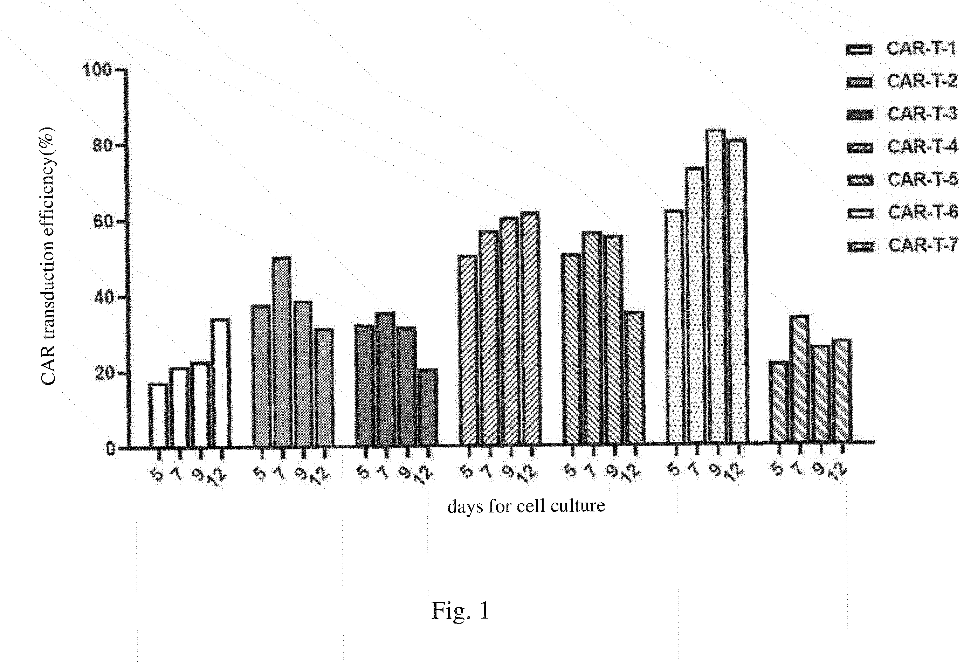

Fig. 1 shows the CAR molecular transduction efficiency of each group of cells after T cell was infected with lentivirus for 5 days, 7 days, 9 days, and 12 days. The CAR molecular transduction efficiency of CAR-T-2, CAR-T-3 and CAR-T-7 groups was close to that of the control group CAR-T-1, and the CAR molecular transduction efficiency of CAR-T-4, CAR-T-5 and CAR-T-6 groups was significantly higher than that of the control group CAR-T-1, indicating that each group of CAR-T cells all showed good CAR protein expression.

### Example 2: In vitro proliferation ability of the modified CD123 CAR-T

The present example took the detection for the proliferation ability of CD123 CAR-T having different antigen recognition domains as an example.

In the CD123 CAR-T cell culture process, the day on T cells infected with lentivirus was taken as the second day of culture, after which every 2 to 3 days, CAR-T cells were collected and centrifuged, 1-2 ml of culture system was added to resuspend, 10 µl of cells were taken and diluted into a certain fold, stained with trypan blue (Solarbio, Cat #C0040), counted for the total number of living cells and the total number of dead cells under an inverted microscope (Ts2-FL, Nikon, Japan), and the proliferation of cells in each group was calculated .

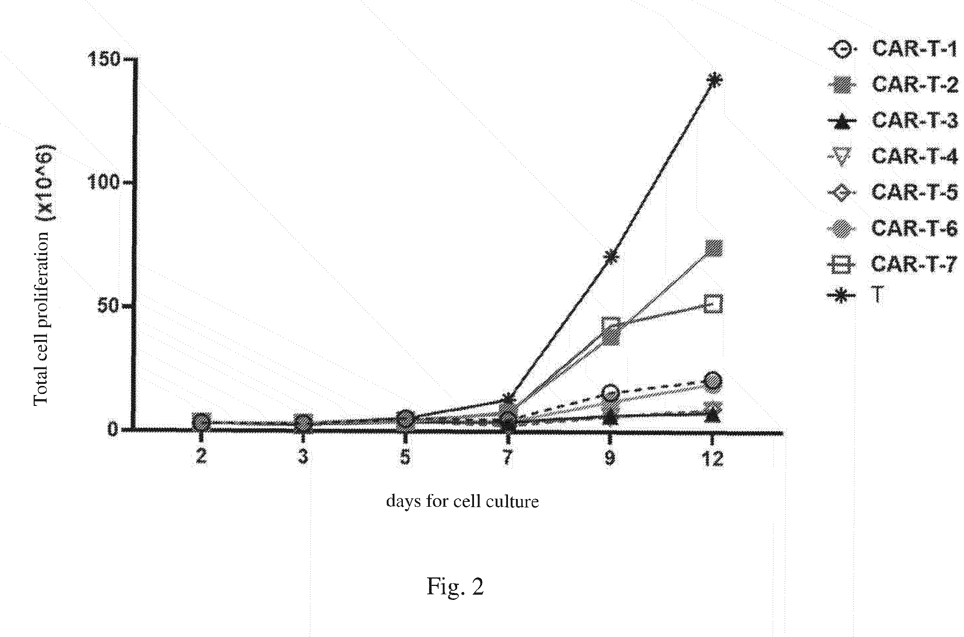

Fig. 2 shows that the total cell proliferation of CAR-T-2 and CAR-T-7 with different antigen recognition domains was significantly improved as compared to the control group CAR-T-1. This result showed that the scFv design of CAR-T-2 and CAR-T-7 can improve the proliferation ability of CD123 CAR-T cells.

### Example 3: Cell differentiation of the modified CD123 CAR-T under target cell stimulation

The present example took the detection for T cell differentiation of CD123 CAR-T having different antigen recognition domains under the stimulation of two target cells AML primary cells or TTP-1 cells (both expressing CD123), respectively, as an example.

When CAR-T cells were cultured to day 5, two portions of 5×10⁵ cells were taken from each group and added to a 12-well plate, and the target cell AML primary cell or THP-1 cell was added to the 12-well plate at the an effector/target ratio (E: T) = 5:1, respectively. After target cell stimulation for 6 days, that is, when CAR-T cells were cultured to day 11, 1×10⁶ cells were taken from each group, centrifuged and resuspended with 100 µl of DPBS (HyClone, CAT#SH30028.02); each group of CAR-T cells is then labelled with T cell differentiation-related fluorescent antibody and detected using a full-spectrum flow cytometer (N7-00008-0A, Cytek Biosciences, Inc. Fremont).

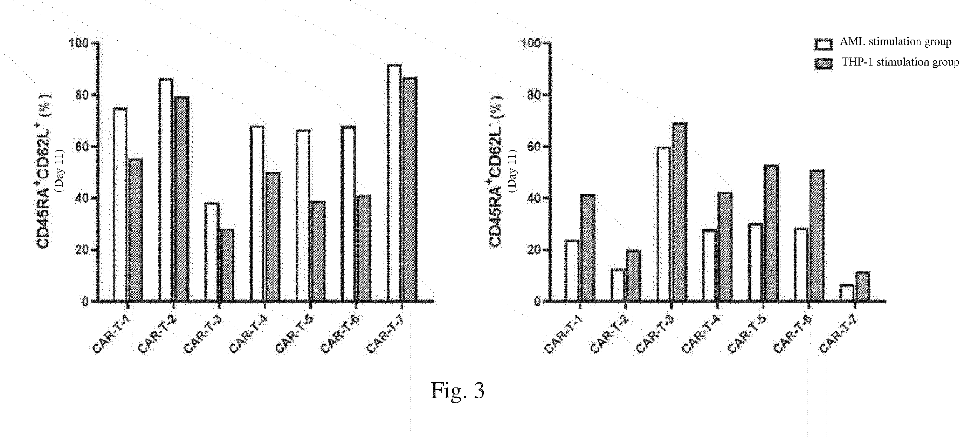

Fig. 3 showed the results, wherein CD45RA⁺CD62L⁺ cell population represented a combination of the initial T cell population and the dry memory T cell population, CD45RA⁺CD62L⁻ cell population was the effector T cell population. The proportion of CD45RA⁺CD62L⁺ cell population of CAR-T-2 and CAR-T-7 with different antigen recognition domains was higher than that of the control group CAR-T-1, and the proportion of CD45RA⁺CD62L⁻ cell population was lower than that of the control group CAR-T-1. This result showed that the modified CD123 antigen recognition domain had better CAR-T cell persistence.

### Example 4: The killing efficiency for the modified CD123 CAR-T against the cell line in vitro

The present example took the detection for killing efficiency of CD123 CAR-T with different antigen recognition domains against tumor cell lines expressing CD123 and those not expressing CD123, as an example.

Cell line THP-1 expressing CD123 and cell line K562 not expressing CD123 were collected and centrifuged, respectively, resuspended with 1 ml of normal saline (Hebei Tiancheng Pharmaceutical Co., Ltd., China), add 5 µl of Calcein-AM (1 µg/µL of concentration, Cat#C3100MP, ThermoFisher, USA), mixed gently, and incubated in a 37 °C incubator for 30 min to label target cells. After the incubation was complete, washed 2 times with normal saline, and X-VIVO 15 was added to resuspend and count. 1×10⁵ of the above-labelled target cells per well were added to 48-well cell culture plate (Corning Incorporated, Corning, NY, USA), each group of CAR-T cells was added to the 48-well cell culture plate at the ratio of E:T = 10:1, and the 48-well culture plate was placed at 37 °C, 5% CO₂ incubator for 5 h of incubation. After the incubation was complete, 2% concentration of Triton-X-100 (Cat#T8787-100ML, Sigma, Germany) was added to lyse a positive control group, i.e., 1×10⁵ of the above-labelled target cells; 100 µl of the killing system supernatant from each well was taken and placed in the microplate plate, and the fluorescence value (excitation wavelength: 495 nm, emission wavelength: 515 nm) was detected by using a multifunctional microplate reader (Varioscan Lux, ThermoFisher).

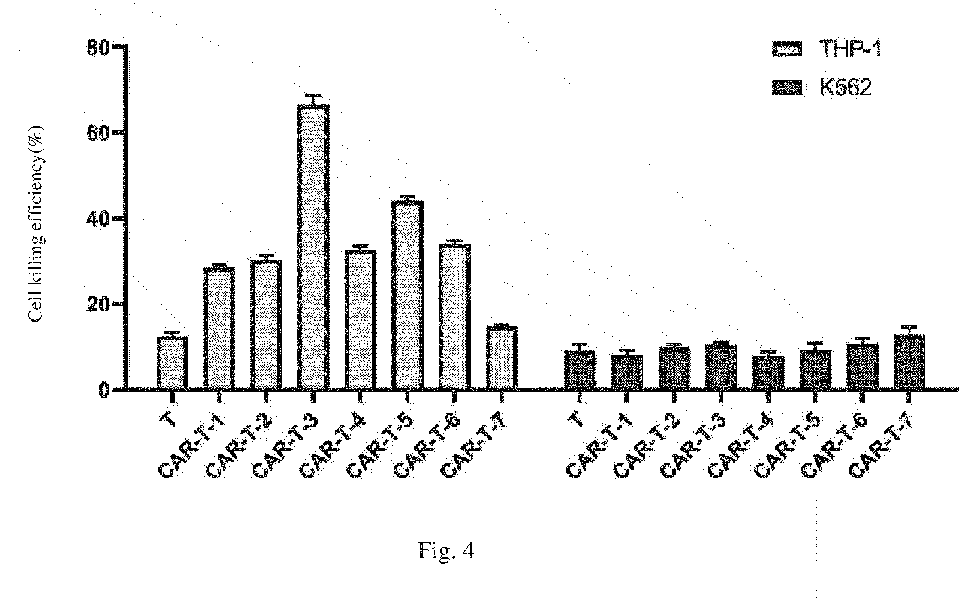

Fig. 4 showed that as for the CD123-positive cell line TRP-1, the killing efficiency of CAR-T-3 and CAR-T-5 in CD123 CAR-T-T with different antigen recognition domains was significantly higher than that of the control group CAR-T-1. Meanwhile, the killing efficiency of each group of modified CD123 CAR-T and control group CAR-T-1 against K562 cell lines not expressing CD123, was consistent with that of the T cell group. This result showed that each group of CD123 CAR-T cells had a certain killing effect on target cells and had good specificity, and the scFv design of CAR-T-3 and CAR-T-5 was more conducive to CAR-T killing tumor cells.

### Example 5: The killing efficiency of the modified CD123 CAR-T against AML primary cells in vitro

The present example took the detection for the killing efficiency of CD123 CAR-T with different antigen recognition domains against AML primary cells as an example.

Three bone marrow blood samples from CD123-positive AML patients with high antigen density (i.e., AML 1#, CD123 expression rate of 99.87%, MFI of 13298), medium antigen density (i.e., AML 2#, CD123 expression rate of 75.4%, MFI of 7518), low antigen density (i.e., AML 3#, CD123 expression rate of 94.58%, MFI of 5252) (antigen density level was defined as follows: high antigen density: median fluorescence intensity (MFI) greater than 10,000; medium antigen density: MFI greater than 6,000 and less than 10,000; low antigen density: MFI less than 6,000) were taken, mononuclear cell was isolated from bone marrow blood samples from AML patients and CD34-positive AML primary cell was sorted using CD34 MicroBeads (Cat#130-100-453, Miltenyi, Germany), resuspended with 1 ml of normal saline, 10 µl of Callein-AM (1 µg/µL of concentration, ThermoFisher, USA) was added, mixed gently, placed a 37 °C incubator for 30 min of incubation to label AML primary cells. After the incubation was complete, washed 2 times with normal saline, and X-VIVO15 was added to resuspend and count. 1×10⁵ of the above-labelled AML primary cells per well were added to 48-well cell culture plate (Corning Incorporated, Corning, NY, USA), CD123 CAR-T cells was added to the 48-well cell culture plate at the ratio of E: T = 10:1, respectively, and the 48-well cell culture plate was placed at 37 °C, 5% CO₂ incubator for 5 h of incubation. After the incubation was complete, 2% concentration of Triton-X-100 lysis positive control group, i.e. 1×10⁵ of the above-labelled AML primary cells, were added; 100 µl of killing system supernatant per well was taken and placed in the microplate plate, and the fluorescence value (excitation wavelength: 495 nm, emission wavelength: 515 nm) was detected using a multifunctional microplate reader (Varioscan Lux, ThermoFisher).

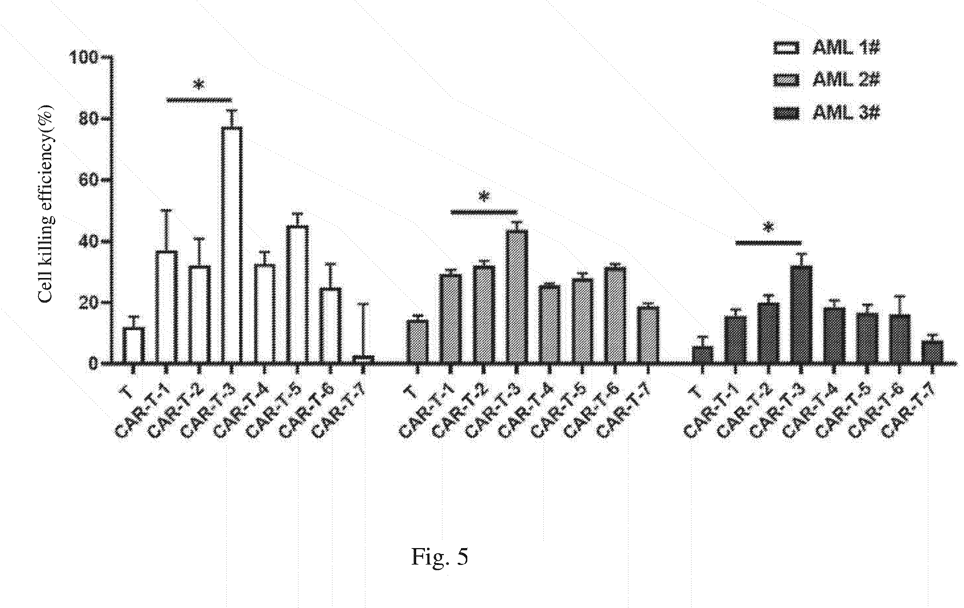

Fig. 5 showed that CAR-T-3 with different antigen recognition domains had a significantly enhanced killing efficiency against three primary cells of CD123-positive AML patients with high (i.e., AML 1#), medium (i.e., AML 2#), and low (i.e., AML 3#) antigen density compared with the control group CAR-T-1. This result suggested that CD123 CAR-T cells with CAR-T-3 scFv can effectively improve the killing efficiency against primary cells.

### Example 6: Cytokine expression of the modified CD123 CAR-T under target cell stimulation

The present example took the detection for the cytokine expression level of CD123 CAR-T with different antigen recognition domains against AML primary cells as an example.

1×10⁵ of target cells AML primary cells per well were added to 48-well cell culture plate (Corning Incorporated, Corning, NY, USA), wherein the CD123 expression rate of the AML primary cells was 99.87% and the MFI was 13298, and each group of CAR-T cells was added to the 48-well cell culture plate at the ratio of E:T=10:1, and the 48-well cell culture plate was placed at 37 °C and 5% CO₂ incubator for 5 h of incubation. The cell supernatant was taken and the expression levels of various cytokines were detected by using CBA kit (BD Biosciences, San Jose, CA, USA) and flow cytometry (NovoCyte 2060R, ACEA Biosciences, San Diego, CA, USA).

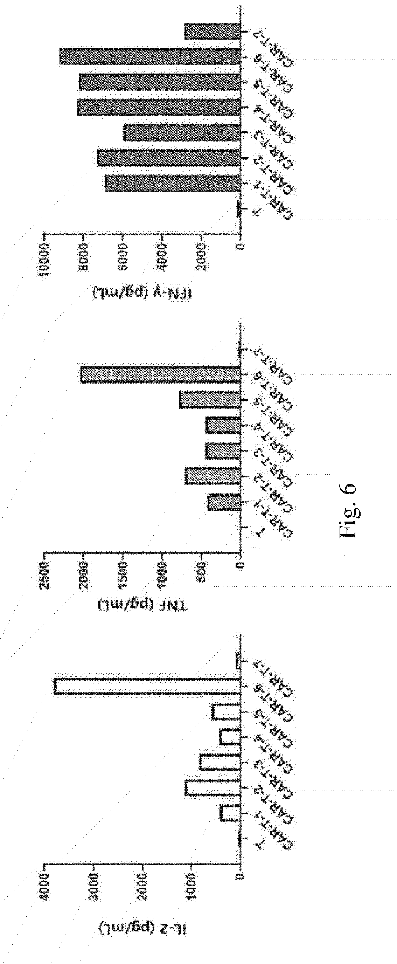

Fig. 6 showed that the expression levels of IL-2, TNF, IFN-γ were significantly improved under target cells stimulation in the CAR-T-6 group with different antigen recognition domains compared with the control group CAR-T-1; and cytokine expression of the remaining groups of CAR-T cells was similar to that of the control group CAR-T-1. This result showed that the scFv design of CAR-T-6 was conducive to the expression of CD123 CAR-T cytokines.

The specific amino acid sequences involved in the present invention are described in the following Tables 1 and 2.

**Table 1 CDR and scFv sequences of exemplary antibodies**

| Clone ID. | | SEQ ID NO | The Amino Acid Sequence |
|---|---|---|---|
| scFv-01 | VH-CDR1 | 7 | GYTFTSYWMN |
| | VH-CDR2 | 13 | RIDPYD SETH YNQKFKDK A |
| | VH-CDR3 | 19 | ARGNWDDY |
| | VL-CDR1 | 25 | ASKSISKDLAW |
| | VL-CDR2 | 31 | GSTLQSG |
| | VL-CDR3 | 37 | QHNKYPYTF |
| | VH | 43 | QVQLQQPGAELVRPGASVKLSCKASGYTFTSYWMNWVKQRPDQGLEWIGRIDPY DSETHYNQKFKDKAILTVDKSSSTAYMQLSSLTSEDSAVYYCARGNWDDYWGQG TTLTVSS |
| | VL | 49 | DVQITQSPSYLAASPGETITINCRASKSISKDLAWYQEKPGKTNKLLIYSGSTLQSG IPSRFSGSGSGTDFTLTISSLEPEDFAMYYCQQHNKYPYTFGGGTKLEIK |
| | scFv | 55 | QVQLQQPGAELVRPGASVKLSCKASGYTFTSYWMNWVKQRPDQGLEWIGRIDPY DSETHYNQKFKDKAILTVDKSSSTAYMQLSSLTSEDSAVYYCARGNWDDYWGQG TTLTVSSGGGGSGGGGSGGGGSDVQITQSPSYLAASPGETITINCRASKSISKDLAW YQEKPGKTNKLLIYSGSTLQSGIPSRFSGSGSGTDFTLTISSLEPEDFAMYYCQQHNK YPYTFGGGTKLEIK |
| scFv-02 | VH-CDR1 | 8 | GYSFTDYYVH |
| | VH-CDR2 | 14 | YISCYNGATNYNPKFKGKA |
| | VH-CDR3 | 20 | AGGEGYYGYDGYAMDY |
| | VL-CDR1 | 26 | ASQDINKYIAW |
| | VL-CDR2 | 32 | TSTLQPG |
| | VL-CDR3 | 38 | QYDDLYTF |
| | VH | 44 | QVQLKQSGPELVKTGASVKMSCKASGYSFTDYYVHWVRQSHGKSLEWIGYISCY NGATNYNPKFKGKATFTVDTSSSTAYMQFKSLTSEDSAVYYCAGGEGYYGYDGYA MDYWGQGTSVTVSS |
| | VL | 50 | DIQLTQSPSSLSASLGGKVTITCKASQDINKYIAWYQHKPGKGPRLLIHYTSTLQPGI PSRFSGSGSGGDYSFSISNLEPEDIATYYCLQYDDLYTFGGGTKLEIK |
| | scFv | 56 | QVQLKQSGPELVKTGASVKMSCKASGYSFTDYYVHWVRQSHGKSLEWIGYISCY NGATNYNPKFKGKATFTVDTSSSTAYMQFKSLTSEDSAVYYCAGGEGYYGYDGYA MDYWGQGTSVTVSSGGGGSGGGGSGGGGSDIQLTQSPSSLSASLGGKVTITCKAS QDINKYIAWYQHKPGKGPRLLIHYTSTLQPGIPSRFSGSGSGGDYSFSISNLEPEDIAT YYCLQYDDLYTFGGGTKLEIK |
| scFv-03 | VH-CDR1 | 9 | GYTFMTYVIH |
| | VH-CDR2 | 15 | YCNPYNDGINYNEKFKGKA |
| | VH-CDR3 | 21 | ARSPSYYGRSYYYGMDY |
| | VL-CDR1 | 27 | ASQSVDYDGDSYMNW |
| | VL-CDR2 | 33 | ASNLESG |
| | VL-CDR3 | 39 | QSNEDPYTF |
| | VH | 45 | QVQLKQSGPELVKPGASVKMSCKASGYTFMTYVIHWVKQKPGQGLEWFGYCNP YNDGINYNEKFKGKATLTSDKSSSTVYMELSSLTSEDSAVYYCARSPSYYGRSYYY GMDYWGQGTSVTVSS |
| | VL | 51 | DIQLTQSPASLAVSLGQRATISCKASQSVDYDGDSYMNWYQQKPGQPPKVLIYAAS NLESGIPARFSGSGSGTDFTLNIHPVEEEDAATYYCQQSNEDPYTFGGGTKLDIK |
| | scFv | 57 | QVQLKQSGPELVKPGASVKMSCKASGYTFMTYVIHWVKQKPGQGLEWFGYCNP |
| | | | YNDGINYNEKFKGKATLTSDKSSSTVYMELSSLTSEDSAVYYCARSPSYYGRSYYY GMDYWGQGTSVTVSSGGGGSGGGGSGGGGSDIQLTQSPASLAVSLGQRATISCKA SQSVDYDGDSYMNWYQQKPGQPPKVLIYAASNLESGIPARFSGSGSGTDFTLNIHP VEEEDAATYYCQQSNEDPYTFGGGTKLDIK |
| scFv-04 | VH-CDR1 | 10 | GYSITSDYAWN |
| | VH-CDR2 | 16 | YISYSGSSNSNPSLKSRI |
| | VH-CDR3 | 22 | ARGMDY |
| | VL-CDR1 | 28 | ASSSVSSSYLHW |
| | VL-CDR2 | 34 | TSNLASG |
| | VL-CDR3 | 40 | QFHRSPRTF |
| | VH | 46 | DVKLQESGPGLVKPSQSLSLTCTVTGYSITSDYAWNWIRQFPGNKLEWMGYISYSG SSNSNPSLKSRI SITRDTSKNQFFLQLNSVTTEDTATYYCARGMDYWGQGTSVTVSS |
| | VL | 52 | DIVLTQSPAIMSVFLGERVTMTCTASSSVSSSYLHWYQQKPGSSPKLWIYSTSNLAS GVPARFSGSGSGTSYSLTISSMEAEDAATYYCHQFHRSPRTFGGGTKLEIK |
| | scFv | 58 | DVKLQESGPGLVKPSQSLSLTCTVTGYSITSDYAWNWIRQFPGNKLEWMGYISYSG SSNSNPSLKSRISITRDTSKNQFFLQLNSVTTEDTATYYCARGMDYWGQGTSVTVS SGGGGSGGGGSGGGGSDIVLTQSPAIMSVFLGERVTMTCTASSSVSSSYLHWYQQK PGSSPKLWIYSTSNLASGVPARFSGSGSGTSYSLTISSMEAEDAATYYCHQFHRSPRT FGGGTKLEIK |
| scFv-05 | VH-CDR1 | 11 | GYTFSSYNMH |
| | VH-CDR2 | 17 | YIYPGNGGTNYNQKFKGKA |
| | VH-CDR3 | 23 | ARERGLYDYDETGYYAMDY |
| | VL-CDR1 | 29 | ASQSISDYLHW |
| | VL-CDR2 | 35 | ASQSISE |
| | VL-CDR3 | 41 | NGHSEPYTF |
| | VH | 47 | QVQLKQSGAELVRSGASVKMSCKASGYTFSSYNMHWVKQTPGQGLEWIGYIYPG NGGTNYNQKFKGKATLTADTSSSTAYMQISSLTSEDSAVYFCARERGLYDYDETGY YAMDYWGQGTSVTVSS |
| | VL | 53 | DIQLTQSPATLSVTPGDRVSLSCRASQSISDYLHWYQQKSHESPRLLIKYASQSISE IPSRFSGSGSGSDFTLSINSVEPEDVGVYFCQNGHSFPYTFGGGTKLEIK |
| | scFv | 59 | QVQLKQSGAELVRSGASVKMSCKASGYTFSSYNMHWVKQTPGQGLEWIGYIYPG NGGTNYNQKFKGKATLTADTSSSTAYMQISSLTSEDSAVYFCARERGLYDYDETGY YAMDYWGQGTSVTVSSGGGGSGGGGS GGGGSDIQLTQSPATLSVTPGDRVSLSCR ASQSISDYLHWYQQKSHESPRLLIKYASQSISEIPSRFSGSGSGSDFTLSINSVEPEDV GVYFCQNGHSFPYTFGGGTKLEIK |
| scFv-06 | VH-CDR1 | 12 | GYSFTGYYMH |
| | VH-CDR2 | 18 | RINPYNNATSYNQNFKDKA |
| | VH-CDR3 | 24 | ARGEGWLLPLAC |
| | VL-CDR1 | 30 | SSQSLLNSSNQKNYLAW |
| | VL-CDR2 | 36 | ASTRDSG |
| | VL-CDR3 | 42 | QHYSTPWTF |
| | VH | 48 | QVQLKQSGPELVKSGASVKISCKASGYSFTGYYMHWVKQSHVKSLEWIGRINPYN NATSYNQNFKDKASLTVDKSSSTAYMELHSLTSEDSAVYYCARGEGWLLPLACWG QGTLVTVSA |
| | VL | 54 | DIVMTQSPSSLAMSVGQKVTMSCKSSQSLLNSSNQKNYLAWYQQKPGQSPKLLV YFASTRDSGVPDRFIGSGSGTDFTLTISSVQAEDLADYFCQQHYSTPWTFGGGTKLE IK |
| | scFv | 60 | QVQLKQSGPELVKSGASVKISCKASGYSFTGYYMHWVKQSHVKSLEWIGRINPYN NATSYNQNFKDKASLTVDKSSSTAYMELHSLTSEDSAVYYCARGEGWLLPLACWG QGTLVTVSAGGGGSGGGGSGGGGSDIVMTQSPSSLAMSVGQKVTMSCKSSQSLL NSSNQKNYLAWYQQKPGQSPKLLVYFASTRDSGVPDRFIGSGSGTDFTLTISSVQA EDLADYFCQQHYSTPWTFGGGTKLEIK |
| scFv-07 | VH-CDR1 | 8 | GYSFTDYYVH |
| | VH-CDR2 | 14 | YISCYNGATNYNPKFKGKA |
| | VH-CDR3 | 20 | AGGEGYYGYDGYAMDY |
| | VL-CDR1 | 26 | ASQDINKYIAW |
| | VL-CDR2 | 32 | TSTLQPG |
| | VL-CDR3 | 38 | QYDDLYTF |
| | VH | 44 | QVQLKQSGPELVKTGASVKMSCKASGYSFTDYYVHWVRQSHGKSLEWIGYISCY NGATNYNPKFKGKATFTVDTSSSTAYMQFKSLTSEDSAVYYCAGGEGYYGYDGYA MDYWGQGTSVTVSS |
| | VL | 50 | DIQLTQSPSSLSASLGGKVTITCKASQDINKYIAWYQHKPGKGPRLLIHYTSTLQPGI PSRFSGSGSGGDYSFSISNLEPEDIATYYCLQYDDLYTFGGGTKLEIK |
| | scFv | 61 | DIQLTQSPSSLSASLGGKVTITCKASQDINKYIAWYQHKPGKGPRLLIHYTSTLQPGI |
| | | | PSRFSGSGSGGDYSFSISNLEPEDIATYYCLQYDDLYTFGGGTKLEIKGGGGSGGGG SGGGGSQVQLKQSGPELVKTGASVKMSCKASGYSFTDYYVHWVRQSHGKSLEWI GYISCYNGATNYNPKFKGKATFTVDTSSSTAYMQFKSLTSEDSAVYYCAGGEGYY GYDGYAMDYWGQGTSVTVSS |

**Table 2 Exemplary chimeric antigen receptor sequences**

| Clone ID | SEQ ID NO | The Amino Acid Sequence |
|---|---|---|
| 4-1BB | 62 | KRGRKKLLYIFKQPFMRPVQTTQEEDGCSCRFPEEEEGGCEL |
| CD3ζ | 63 | RVKFSRSADAPAYKQGQNQLYNELNLGRREEYDVLDKRRGRDPEM GGKPRRKNPQEGLYNELQKDKMAEAYSEIGMKGERRRGKGHDG LYQGLSTATKDTYDALHMQALPPR |
| CAR-1 | 64 | MLLLVTSLLLCELPHPAFLLIPNWSHPQFEKGGGGSGGGGSQVQLQQ PGAELVRPGASVKLSCKASGYTFTSYWMNWVKQRPDQGLEWIGRID PYDSETHYNQKFKDKAILTVDKSSSTAYMQLSSLTSEDSAVYYCARGN WDDYWGQGTTLTVSSGGGGSGGGGSGGGGSDVQITQSPSYLAASPG ETITINCRASKSISKDLAWYQEKPGKTNKLLIYSGSTLQSGIPSRFSGSG SGTDFTLTISSLEPEDFAMYYCQQHNKYPYTFGGGTKLEIKTTTPAPRP PTPAPTIASQPLSLRPEACRPAAGGAVHTRGLDFACDIYIWAPLAGTCG VLLLSLVITLYCKRGRKKLLYIFKQPFMRPVQTTQEEDGCSCRFPEEEE GGCELRVKFSRSADAPAYKQGQNQLYNELNLGRREEYDVLDKRRGRD PEMGGKPRRKNPQEGLYNELQKDKMAEAYSEIGMKGERRRGKGHDG LYQGLSTATKDTYDALHMQALPPR |
| CAR-2 | 65 | MLLLVTSLLLCELPHPAFLLIPNWSHPQFEKGGGGSGGGGSQVQLKQS GPELVKTGASVKMSCKASGYSFTDYYVHWVRQSHGKSLEWIGYISCY NGATNYNPKFKGKATFTVDTSSSTAYMQFKSLTSEDSAVYYCAGGEGY YGYDGYAMDYWGQGTSVTVSSGGGGSGGGGSGGGGSDIQLTQSPSSL SASLGGKVTITCKASQDINKYIAWYQHKPGKGPRLLIHYTSTLQPGIPSR FSGSGSGGDYSFSISNLEPEDIATYYCLQYDDLYTFGGGTKLEIKTTTPAP RPPTPAPTIASQPLSLRPEACRPAAGGAVHTRGLDFACDIYIWAPLAGTCG VLLLSLVITLYCKRGRKKLLYIFKQPFMRPVQTTQEEDGCSCRFPEEEEG GCELRVKFSRSADAPAYKQGQNQLYNELNLGRREEYDVLDKRRGRDPE MGGKPRRKNPQEGLYNELQKDKMAEAYSEIGMKGERRRGKGHDGLYQ GLSTATKDTYDALHMQALPPR |
| CAR-3 | 66 | MLLLVTSLLLCELPHPAFLLIPNWSHPQFEKGGGGSGGGGSQVQLKQSG PELVKPGASVKMSCKASGYTFMTYVIHWVKQKPGQGLEWFGYCNPYN DGINYNEKFKGKATLTSDKSSSTVYMELSSLTSEDSAVYYCARSPSYYGR SYYYGMDYWGQGTSVTVSSGGGGSGGGGSGGGGSDIQLTQSPASLAVS LGQRATISCKASQSVDYDGDSYMNWYQQKPGQPPKVLIYAASNLESGIP ARFSGSGSGTDFTLNIHPVEEEDAATYYCQQSNEDPYTFGGGTKLDIKTT TPAPRPPTPAPTIASQPLSLRPEACRPAAGGAVHTRGLDFACDIYIWAPLAG TCGVLLLSLVITLYCKRGRKKLLYIFKQPFMRPVQTTQEEDGCSCRFPEEE EGGCELRVKFSRSADAPAYKQGQNQLYNELNLGRREEYDVLDKRRGRDPE MGGKPRRKNPQEGLYNELQKDKMAEAYSEIGMKGERRRGKGHDGLYQG LSTATKDTYDALHMQALPPR |
| CAR-4 | 67 | MLLLVTSLLLCELPHPAFLLIPNWSHPQFEKGGGGSGGGGSDVKLQESGPG LVKPSQSLSLTCTVTGYSITSDYAWNWIRQFPGNKLEWMGYISYSGSSNSNP SLKSRISITRDTSKNQFFLQLNSVTTEDTATYYCARGMDYWGQGTSVTVSSG GGGSGGGGSGGGGSDIVLTQSPAIMSVFLGERVTMTCTASSSVSSSYLHWYQ QKPGSSPKLWIYSTSNLASGVPARFSGSGSGTSYSLTISSMEAEDAATYYCHQ FHRSPRTFGGGTKLEIKTTTPAPRPPTPAPTIASQPLSLRPEACRPAAGGAVHT RGLDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERR RGKGHDGLYQGLSTATKDTYDALHMQALPPR |
| CAR-5 | 68 | MLLLVTSLLLCELPHPAFLLIPNWSHPQFEKGGGGSGGGGSQVQLKQSGAEL VRSGASVKMSCKASGYTFSSYNMHWVKQTPGQGLEWIGYIYPGNGGTNYN QKFKGKATLTADTSSSTAYMQISSLTSEDSAVYFCARERGLYDYDETGYYAMD YWGQGTSVTVSSGGGGSGGGGSGGGGSDIQLTQSPATLSVTPGDRVSLSCRAS QSISDYLHWYQQKSHESPRLLIKYASQSISEIPSRFSGSGSGSDFTLSINSVEPED VGVYFCQNGHSFPYTFGGGTKLEIKTTTPAPRPPTPAPTIASQPLSLRPEACRPA AGGAVHTRGLDFACDIYIWAPLAGTCGVLLLSLVITLYCKRGRKKLLYIFKQPF |
| | | MRPVQTTQEEDGCSCRFPEEEEGGCELRVKFSRSADAPAYKQGQNQLYNELN LGRREEYDVLDKRRGRDPEMGGKPRRKNPQEGLYNELQKDKMAEAYSEIGM KGERRRGKGHDGLYQGLSTATKDTYDALHMQALPPR |
| CAR-6 | 69 | MLLLVTSLLLCELPHPAFLLIPNWSHPQFEKGGGGSGGGGSQVQLKQSGPELV KSGASVKISCKASGYSFTGYYMHWVKQSHVKSLEWIGRINPYNNATSYNQNF KDKASLTVDKSSSTAYMELHSLTSEDSAVYYCARGEGWLLPLACWGQGTLVT VSAGGGGSGGGGSGGGGSDIVMTQSPSSLAMSVGQKVTMSCKSSQSLLNSSN QKNYLAWYQQKPGQSPKLLVYFASTRDSGVPDRFIGSGSGTDFTLTISSVQAED LADYFCQQHYSTPWTFGGGTKLEIKTTTPAPRPPTPAPTIASQPLSLRPEACRPA AGGAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYNELNL GRREEYDVLDKRRGRDPEMGGKPRRKNPQEGLYNELQKDKMAEAYSEIGMK GERRRGKGHDGLYQGLSTATKDTYDALHMQALPPR |
| CAR-7 | 70 | MLLLVTSLLLCELPHPAFLLIPNWSHPQFEKGGGGSGGGGSDIQLTQSPSSLSAS LGGKVTITCKASQDINKYIAWYQHKPGKGPRLLIHYTSTLQPGIPSRFSGSGSGG DYSFSISNLEPEDIATYYCLQYDDLYTFGGGTKLEIKGGGGSGGGGSGGGGSQV QLKQSGPELVKTGASVKMSCKASGYSFTDYYVHWVRQSHGKSLEWIGYISCYN GATNYNPKFKGKATFTVDTSSSTAYMQFKSLTSEDSAVYYCAGGEGYYGYDGY AMDYWGQGTSVTVSSTTTPAPRPPTPAPTIASQPLSLRPEACRPAAGGAVHTRGL DFACDIYIWAPLAGTCGVLLLSLVITLYCKRGRKKLLYIFKQPFMRPVQTTQEEDG CSCRFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGLYQ GLSTATKDTYDALHMQALPPR |

References:
[1] Surveillance, Epidemiology, and End Results (SEER) Program. Research Data (1973-2015), National Cancer Institute, Deeps, Surveillance Research Program, Released April 2018, Based on the November 2017 Submission. 2019. (www.seer.cancer.gov).
[2] Thein MS, Ershler WB, Jemal A, Yates JW, Baer MR. Outcome of older patients with acute myeloid leukemia: an analysis of SEER data over 3 decades. Cancer 2013;119(15):2720-7.
[3] Kantarjian HM, DiNardo CD, Nogueras-Gonzalez GM, Kadia TM, Jabbour E, Bueso-Ramos CE, et al. Results of second salvage therapy in 673 adults with acute myelogenous leukemia treated at a single institution since 2000. Cancer 2018;124(12):2534-40.
[4] Jang J, Lee J, Jang JH, Jung CW, Park S. Anti-leukemic effects of simvastatin on NRAS(G12D) mutant acute myeloid leukemia cells. Mol Biol Rep. 2019;46:5859-66.
[5] Roboz GJ. Novel approaches to the treatment of acute myeloid leukemia. Hematology Am Soc Hematol Educ Program. 2011;2011:43-50.
[6] Benmebarek, M.R.; Karches, C.H.; Cadilha, B.L.; Lesch, S.; Endres, S.; Kobold, S. Killing mechanisms of chimeric antigen receptor (CAR) T cells. Int. J. Mol. Sci. 2019, 20, 1283.
[7] Gross, G.; Waks, T.; Eshhar, Z. Expression of immunoglobulin-T-cell receptor chimeric molecules as functional receptors with antibody-type specificity. Proc. Natl. Acad. Sci. USA 1989, 86, 10024-10028.
[8] Khalil DN, Smith EL, Brentjens RJ, Wolchok JD. The future of cancer treatment: immunomodulation, CARs and combination immunotherapy. Nat Rev Clin Oncol 2016;13(6):394.
[9] Muñoz L, Nomdedéu JF, López O, et al. Interleukin-3 receptor α chain (CD123) is widely expressed in hematologic malignancies. Haematologica. 2001;86:1261-9.
[10] Blalock WL, Weinstein-Oppenheimer C, Chang F, et al. Signal transduction, cell cycle regulatory, and anti-apoptotic pathways regulated by IL-3 in hematopoietic cells: possible sites for intervention with anti-neoplastic drugs. Leukemia Aug. 1999;13:1109-66.
[11] Testa U, Riccioni R, Militi S, et al. Elevated expression of IL-3Ralpha in acute myelogenous leukemia is associated with enhanced blast proliferation, increased cellularity, and poor prognosis. Blood. 2002;100:2980-8.
[12] Jordan CT, Upchurch D, Szilvassy SJ, et al. The interleukin-3 receptor alpha chain is a unique marker for human acute myelogenous leukemia stem cells. Leukemia, 2000,14(10), 1777-1784.
[13] Wognum AW, De Jong MO, Wagemaker G. Differential Expression of Receptors for Hemopoietic Growth Factors on Subsets of CD34+Hemopoietic Cells. Leukemia & Lymphoma,1996, 24(1-2), 11-25.
[14] Huang S, Chen Z, Yu JF, et al. Correlation Between IL - 3 Receptor Expression and Growth Potential of Human CD34+Hematopoietic Cells from Different Tissues. Stem Cells, 1999,17(5), 265-272.
[15] Munoz L, Nomdedeu JF, Lopez O, et al. Interleukin-3 Receptor Alpha Chain (CD123) Is Widely Expressed In Hematologic Malignancies. Haematologica, 2001 86: 1261-1269
[16] Xie LH, Biondo M, Busfield SJ, et al. CD123 target validation and preclinical evaluation of ADCC activity of anti-CD123 antibody CSL362 in combination with NKs from AML patients in remission. Blood Cancer Journal, 2017,7(6), e567.
[17] Baroni ML, Sanchez Martinez D, Gutierrez Aguera F, et al. (2020). 41BB-based and CD28-based CD123-redirected T-cells ablate human normal hematopoiesis in vivo. Journal for ImmunoTherapy of Cancer, 2020,8(1), e000845.
[18] Testa U, Pelosi E, Frankel A. CD123 is a membrane biomarker and a therapeutic target in hematologic malignancies. Biomarker Research,2014, 2(1), 4.
[19] Bras AE, de Haas V, van Stigt A, et al. CD123 expression levels in 846 acute leukemia patients based on standardized immunophenotyping. Cytometry Part B: Clinical Cytometry.2018.
[20] Gauthier J, Turtle CJ. Insights into cytokine release syndrome and neurotoxicity after CD19-specific CAR-T cell therapy. Curr Res Transl Med 2018;66(2):50-2.
[21] Cummins K, Frey N, Nelson A, Schmidt A, Luger S, Hexner E, et al. Treating Relapsed/Refractory. (RR) AML With Biodegradable Anti-CD123 CAR Modified T Cells (2017) Atlanta, GA: Blood ASH.

## Claims

1. An antibody that specifically binds to CD123, comprising a heavy chain variable domain VH and a light chain variable domain VL,
wherein the heavy chain variable domain comprises a VH-CDR1 amino acid sequence as shown in SEQ ID NO: 1 (GYX₁X₂X₃X₄(D)YX₅X₆X₇), a VH-CDR2 amino acid sequence as shown in SEQ ID NO: 2 (X₈X₉X₁₀(X₁₁)X₁₂X₁₃X₁₄X₁₅X₁₆X₁₇X₁₈NX₁₉X₂₀X₂₁KX₂₂X₂₃X₂₄) and a VH-CDR3 amino acid sequence as shown in SEQ ID NO: 3 (AX₂₅X₂₆X₂₇X₂₈X₂₉X₃₀X₃₁(YD)X₃₂X₃₃X₃₄X₃₅X₃₆X₃₇X₃₈X₃₉X₄₀);
wherein, X₁ is selected from S and T; X₂ is selected from F and I; X₃ is selected from T, S and M; X₄ is selected from S, D, T and G; X₅ is selected from W, Y, V, A and N; X₆ is selected from M, V, I and W; X₇ is selected from N and H; X₈ is selected from Y and R; X₉ is selected from I and C; X₁₀ is selected from D, N, Y and S; X₁₁ is selected from P and C; X₁₂ is selected from Y and G; X₁₃ is selected from D, N and S; X₁₄ is selected from S, N, D and G; X₁₅ is selected from E, A, G and S; X₁₆ is selected from T, I and S; X₁₇ is selected from H, S and N; X₁₈ is selected from Y and S; X₁₉ is selected from Q, E and P; X₂₀ is selected from K, N and S; X₂₁ is selected from F and L; X₂₂ is selected from D, G and S; X₂₃ is selected from K and R; X₂₄ is selected from A and I; X₂₅ is selected from R and G; X₂₆ is selected from G, S and E; X₂₇ is selected from E, P and R; X₂₈ is selected from G and S; X₂₉ is selected from N, Y, L and W; X₃₀ is selected from W, Y and L; X₃₁ is selected from G and D; X₃₂ is selected from R and E; X₃₃ is selected from Y, S and T; X₃₄ is selected from D, Y and G; X₃₅ is selected from G and Y; X₃₆ is selected from Y and L; X₃₇ is selected from A, G and P; X₃₈ is selected from M and L; X₃₉ is selected from D and A; and X₄₀ is selected from Y and C;
wherein the light chain variable domain comprises VL-CDR1 amino acid sequence as shown in SEQ ID NO: 4 (X₄₁SX₄₂X₄₃X₄₄X₄₅X₄₆X₄₇X₄₈X₄₉(QK)X₅₀X₅₁X₅₂X₅₃W), VL-CDR2 amino acid sequence as shown in SEQ ID NO: 5 (X₅₄SX₅₅X₅₆X₅₇X₅₈X₅₉) and VL-CDR3 amino acid sequence as shown in SEQ ID NO: 6 (X₆₀X₆₁X₆₂X₆₃X₆₄(P) X₆₅TF);
wherein X₄₁ is selected from A and S; X₄₂ is selected from Q, K and S; X₄₃ is selected from S and D; X₄₄ is selected from I, V and L; X₄₅ is selected from S, N, D and L; X₄₆ is selected from K, Y, S, D and N; X₄₇ is selected from D and S; X₄₈ is selected from G and S; X₄₉ is selected from D and N; X₅₀ is selected from S and N; X₅₁ is selected from D and Y; X₅₂ is selected from L, I and M; X₅₃ is selected from A, N and H; X₅₄ is selected from G, T and A; X₅₅ is selected from T, N and Q; X₅₆ is selected from L, S and R; X₅₇ is selected from Q, E, A, I and D; X₅₈ is selected from S and P; X₅₉ is selected from G and E; X₆₀ is selected from Q and N; X₆₁ is selected from H, Y, S, F and G; X₆₂ is selected from N, D, H and Y; X₆₃ is selected from K, L, E, R and S; X₆₄ is selected from Y, L, D, S, F and T; and X₆₅ is selected from Y, R and W;
preferably, the VH-CDR1 comprises an amino acid sequence selected from the group consisting of SEQ ID NOs: 8, 9, 10, 11 and 12; the VH-CDR2 comprises an amino acid sequence selected from the group consisting of SEQ ID NOs: 14, 15, 16, 17 and 18; the VH-CDR3 comprises an amino acid sequence selected from the group consisting of SEQ ID NOs: 20, 21, 22, 23 and 24; the VL-CDR1 comprises an amino acid sequence selected from the group consisting of SEQ ID NOs: 26, 27, 28, 29 and 30; the VL-CDR2 comprises an amino acid sequence selected from the group consisting of SEQ ID NOs: 32, 33, 34, 35 and 36; and the VL-CDR3 comprises an amino acid sequence selected from the group consisting of SEQ ID NOs: 38, 39, 40, 41 and 42;
more preferably, the antibody comprising:
1) a heavy chain variable domain containing VH-CDR1 as shown in SEQ ID NO: 8, VH-CDR2 as shown in SEQ ID NO: 14 and VH-CDR3 as shown in SEQ ID NO: 20; and a light chain variable domain containing VL-CDR1 as shown in SEQ ID NO: 26, VL-CDR2 as shown in SEQ ID NO: 32 and VL-CDR3 as shown in SEQ ID NO: 38; and/or
2) a heavy chain variable domain containing VH-CDR1 as shown in SEQ ID NO: 9, VH-CDR2 as shown in SEQ ID NO: 15 and VH-CDR3 as shown in SEQ ID NO: 21; and a light chain variable domain containing VL-CDR1 as shown in SEQ ID NO: 27, VL-CDR2 as shown in SEQ ID NO: 33 and VL-CDR3 as shown in SEQ ID NO: 39; and/or
3) a heavy chain variable domain containing VH-CDR1 as shown in SEQ ID NO: 10, VH-CDR2 as shown in SEQ ID NO: 16 and VH-CDR3 as shown in SEQ ID NO: 22; and a light chain variable domain containing VL-CDR1 as shown in SEQ ID NO: 28, VL-CDR2 as shown in SEQ ID NO: 34 and VL-CDR3 as shown in SEQ ID NO: 40; and/or
4) a heavy chain variable domain containing VH-CDR1 as shown in SEQ ID NO: 11, VH-CDR2 as shown in SEQ ID NO: 17 and VH-CDR3 as shown in SEQ ID NO: 23; and a light chain variable domain containing VL-CDR1 as shown in SEQ ID NO: 29, VL-CDR2 as shown in SEQ ID NO: 35 and VL-CDR3 as shown in SEQ ID NO: 41; and/or
5) a heavy chain variable domain containing VH-CDR1 as shown in SEQ ID NO: 12, VH-CDR2 as shown in SEQ ID NO: 18 and VH-CDR3 as shown in SEQ ID NO: 24; and a light chain variable domain containing VL-CDR1 as shown in SEQ ID NO: 30, VL-CDR2 as shown in SEQ ID NO: 36 and VL-CDR3 as shown in SEQ ID NO: 42.

2. The antibody according to claim 1, wherein the heavy chain variable domain comprises an amino acid sequence selected from the group consisting of SEQ ID NOs: 44, 45, 46, 47 and 48, or an amino acid sequence having at least 85%, at least 90%, at least 95% or at least 99% identity thereto; and/or
the light chain variable domain comprises an amino acid sequence selected from the group consisting of SEQ ID NOs: 50, 51, 52, 53 and 54, or an amino acid sequence having at least 85%, at least 90%, at least 95% or at least 99% identity thereto;
preferably, the antibody comprises:
1) a heavy chain variable domain as shown in SEQ ID NO: 44 and a light chain variable domain as shown in SEQ ID NO: 50; and/or
2) a heavy chain variable domain as shown in SEQ ID NO: 45 and a light chain variable domain as shown in SEQ ID NO: 51; and/or
3) a heavy chain variable domain as shown in SEQ ID NO: 46 and a light chain variable domain as shown in SEQ ID NO: 52; and/or
4) a heavy chain variable domain as shown in SEQ ID NO: 47 and a light chain variable domain as shown in SEQ ID NO: 53; and/or
5) a heavy chain variable domain as shown in SEQ ID NO: 48 and a light chain variable domain as shown in SEQ ID NO: 54;
more preferably, the antibody comprises an amino acid sequence selected from the group consisting of SEQ ID NOs: 56, 57, 58, 59, 60 and 61, or an amino acid sequence having at least 85%, at least 90%, at least 95% or at least 99% identity thereto.

3. The antibody according to claim 1 or 2, wherein the antibody is a single-chain antibody scFv;
more preferably, the heavy chain variable domain and the light chain variable domain of the single-chain antibody scFv are linked by a linker peptide chain rich in glycine and serine, and the order of the heavy chain variable domain and the light chain variable domain can be interchanged.

4. A chimeric antigen receptor targeting CD123, comprising: the antibody according to any one of claims 1-3; hinge region; transmembrane domain; co-stimulation domain; and CD3ζ intracellular signal transduction domain;
preferably, the co-stimulation domain is selected from CD27, CD28, 4-1BB, OX-40, CD30, CD40, PD-1, ICOS, LFA-1, CD-2, CD7, LIGHT, NKG2C, B7-H3, or any combination thereof; more preferably, the co-stimulation domain is selected from 4-1BB and CD28, and the 4-1BB comprises an amino acid sequence as shown in SEQ ID NO: 62.
preferably, the CD3ζ intracellular signal transduction domain comprises an amino acid sequence as shown in SEQ ID NO: 63;
preferably, the hinge region and the transmembrane domain are selected from IgG1, IgG4, CD8α, CD28, IL-2 receptor, IL-7 receptor, IL-11 receptor, PD-1 or CD34 hinge region and transmembrane domain.

5. The chimeric antigen receptor according to claim 4, wherein the chimeric antigen receptor comprises an amino acid sequence selected from the group consisting of SEQ ID NOs: 65, 66, 67, 68, 69 and 70, or an amino acid sequence having at least 85%, at least 90%, at least 95%, at least 99% identity thereto.

6. A nucleic acid molecule comprising a nucleotide sequence encoding the antibody according to any one of claims 1-3 or the chimeric antigen receptor according to any one of claims 4-5.

7. A vector comprising the nucleic acid molecule according to claim 6;
preferably, the vector is a viral vector;
more preferably, the vector is a lentiviral vector.

8. A cell which is transfected with the vector according to claim 7, thereby comprising the nucleic acid molecule according to claim 6 or the vector according to claim 7;
preferably, the cell is T cell.

9. A pharmaceutical composition comprising the antibody according to any one of claims 1-3, the chimeric antigen receptor according to any one of claims 4-5, the nucleic acid molecule according to claim 6, the vector according to claim 7 or the cell according to claim 8, and a pharmaceutically acceptable carrier.

10. The use of the antibody according to any one of claims 1-3, the chimeric antigen receptor according to any one of claims 4-5, the nucleic acid molecule according to claim 6, the vector according to claim 7 or the cell according to claim 8 in the preparation of a drug for treating tumor expressing CD123;
preferably, the tumor expressing CD123 is acute myeloid leukemia (AML).
